# EUROPEAN PATENT SPECIFICATION

(11) **EP 1 656 963 B1**
(45) Date of publication and mention of the grant of the patent: **21.11.2007**
(21) Application number: 04078086.8
(22) Date of filing: 10.11.2004
(51) Int. Cl.: A61M 25/00, A61F 2/06

(54) **Stent delivery catheter assembly**
Einführkatheteranordnung für stents
Ensemble cathéter de déploiement d'endoprothèse

(43) Date of publication of application: 17.05.2006
(73) Proprietor: Creganna Technologies Limited, Ballybrit, Galway (IE)
(72) Inventor: Fahey, Seamus, Gort County Galway (IE); McDermott, Eamonn, County Leitrim (IE); Farrissey, Liam, Kingston County Galway (IE); Gilbert, John, Oranmore County Galway (IE); Forde, Colin, Kinvara County Galway (IE); Trip, Erik, 5731 NW Mierlo (NL)
(74) Representative: Shortt, Peter Bernard

(56) References cited:
- EP-A- 0 608 853
- EP-A- 0 812 579
- WO-A-03/041783
- US-A- 5 315 996
- US-A- 5 454 787
- US-A- 5 743 876
- US-A- 6 022 343
- US-A- 6 027 863
- US-A1- 2004 117 000
- US-B1- 6 743 219

## Description

### Field of the Invention

The invention relates to systems for the delivery of self-expanding stents or other medical devices to locations within any lumen of the human body, including the vasculature, the biliary system, oesophagus, and gastrointestinal tract.

The invention can also be employed in medical devices that are termed Over The Wire (O.T.W) and Rapid Exchange (Rx.).

### Background to the Invention

Catheters for insertion into bodily lumens, e.g. intravascular catheters and the like are well known in the art. Catheters typically employ elongate flexible tubes made from a synthetic plastics material or from stainless steel. Desirably characteristics of catheter tubing include "pushability", that is the ability to transfer forces from the proximal to the distal end of the catheter. It is also an advantage for the catheter to have good "trackability", i.e. to be sufficiently flexible as to be capable of navigating tortuous paths within a body lumen without kinking.

Small diameter tubing of the kind commonly referred to as "hypotubes" have desirable characteristics in terms of pushability in catheter design, but are sometimes prone to kinking.

Hypotubes are suitable for use with a wide range of catheters, including angioplasty catheters, and stent delivery catheters.

In the field of medical technology, stents are commonly used in angioplasty procedures to widen constricted body lumens. Stents are generally cylindrical in shape and are formed as an open lattice or frame comprising a specific set of meander patterns. These patterns allow the stent to be compressed for delivery through the vasculature of the body to a particular location of constriction or stenosis. The stent is then expanded to exert an outward radial force on the constricted lumen, thereby widening the vessel to counteract the constriction. Stents may be self-expanding, wherein the stent expands once the cause of compression is removed or withdrawn. Alternatively, stents may be expanded by inflation of a balloon catheter within the stent body. This invention is primarily concerned with self-expanding stents.

Self-expanding stents are compressed from a normal, unstressed configuration into a delivery configuration with a reduced diameter for delivery to a constricted location within any lumen of the body. The stent is then released from its delivery system, removing the cause of compression, and is thus allowed to expand into its original uncompressed configuration with its normal diameter. The expansion of the stent exerts an outward force on the walls of the lumen, counteracting the constriction and restoring patency to the vessel.

Delivery systems for self-expanding stents are well known in the relevant field. In general, these comprise a catheter in which a self-expanding stent is disposed. The catheter is directed through the patient's anatomy until the distal end of the catheter has reached the area of constriction. At this point, an outer portion of the catheter is withdrawn, allowing the stent to expand and widen the lumen at the appropriate point.

US-A-2004/0117000 discloses a stent delivery system for delivery of a self-expanding stent to a predetermined location in a body lumen. The delivery system has a catheter body, a retractable outer sheath and a proximal retraction handle connected to a proximal end of a catheter body. The catheter body carries the stent near a distal end of the catheter body for transporting the stent for deployment. The sheath surrounds and contains the stent in a delivery configuration where the stent has a reduced radius along its entire axial length. The sheath has an outer tube and a separate inner tube. The outer tube has a distal end portion surrounding the stent along its entire length and a proximal end portion connected to the retraction handle. The inner tube is disposed concentrically within the outer tube and has a distal end portion surrounding the stent only along a part of its entire length. The outer tube is a co-extruded tubing, e.g. made from a polyamide, whilst the inner tube is made from polyethylene.

US-B-6,743,219 discloses stent delivery apparatus. The apparatus has an outer sheath forming an elongated tubular member having distal and proximal ends and an inside and outside diameter. The apparatus also includes an inner shaft located coaxially within the outer sheath. The inner shaft has a distal end, a proximal end and a longitudinal axis extending therebetween. At least a portion of the inner shaft is made from a flexible coiled member.

The flexible coiled member is a compressed coil spring made from high density polyethylene and nylon, and is capable of stretching and compressing. A proximal end of the apparatus is a stainless steel hypotube. The hypotube is stainless steel and has a 1.067 mm (0.042 inch) outside diameter at its proximal end and then tapers to a 0.914 mm (0.036 inch) outside diameter at its distal end. The inside diameter of the hypotube is 0.813 mm (0,032 inch) throughout its length. The tapered outside diameter is to gradually change the stiffness of the hypotube along its length. This change in the hypotube stiffness is said to allow for a more rigid proximal end or handle end that is needed during stent deployment. If the proximal end is not stiff enough the hypotube section extending beyond the valve could buckle as the deployment forces are transmitted. The distal end of the hypotube is more flexible. The distal end of the hypo also needs to be flexible to minimize the transition between the hypo and the coil section. The outer shaft is a braided hose.

US 6,048,338 discloses a spiral cut transition member for controlling the transition in stiffness of a catheter. The transition member has a spiral cut provided therein to vary the flexibility of the transition member over its length. The pitch of the spiral cut can be varied to facilitate a gradual transition in flexibility along the catheter. Other spiral cut hypotubes are disclosed, for example, in US-B-6,102,890; US-B-5,961,510; US-B-4,960,410 and US-B-5,843,050. The use of a spirally cut stainless steel hypotube of the kind described in the prior art may cause problems in accurately locating a stent, because of inherent axial flexibility and stretch in the tube. None of the prior art documents disclose a stent delivery catheter utilising a stainless steel hypotube, or the use of a pair of co-axially disposed hypotubes in accordance with the present invention.

European Patent Application Publication No. 0 608 853 discloses a vascular dilatation instrument and catheter. US Patent No. 5,454,787 relates to a torquable tubular assembly and torquable catheter utilizing the same. US Patent No. 6,027,863 describes a method for manufacturing a tubular medical device. US Patent No. 5,315,966 describes a torquable catheter and method.

### Object of the Invention

It is an object of the invention to provide a delivery system for a self-expanding stent or any other device to be deployed inside the human body using similar deployment techniques to self expanding stents, examples of such devices are filters staples or self expanding occlusion devices, utilising outer and inner metallic hypotubes, which have improved pushability and flexibility characteristics. It is also an object to provide a delivery system for a self-expanding stent utilising a pair of hypotubes which are spirally or circumferentially cut while ensuring good flexibility and pushability without allowing significant stretching or compression of the tubes.

### Summary of the Invention

The invention relates to a delivery catheter assembly as set out in the appended claims.

According to the invention the delivery catheter assembly for a self-expanding stent, comprises an inner shaft, having proximal and distal ends, and an outer shaft, having proximal and distal ends, and the inner shaft is located coaxially within said outer shaft, and said outer haft extends beyond the distal end of said inner shaft; said distal end of said outer shaft being adapted to accommodate the stent for delivery within a body -lumen-of a patient, characterised in that the inner shaft is an elongate-metallic hypotube and the outer shaft is an elongate metallic hypotube, and each of the hypotubes has a plurality of slots cut in the side wall thereof to increase the flexibility of the hypotube, and the slot are elongate slots extending about the hypotube in a spiral or circumferential path which is interrupted at intervals by solid struts.

The term "hypotube" refers generally to an elongate metallic tube, preferably of stainless steel, having a lumen extending therethrough. The tube is preferably of thin-walled construction. For the present invention, a spiral-cut hypotube may be regarded as a hypotube having at least one cut formed in an outer wall of the tube and extending spirally, e.g. in a helical path, or at an angle to the longitudinal axis of the tube of the hypotube. The spiral-cut hypotubes of the inner and outer shafts provide the delivery catheter of the present invention with improved flexibility over prior art delivery devices. The spiral cut in each hypotube, which may be in the form of a slot or slit, gives the tube a greater degree of flexibility and trackability in all directions. Trackability refers to the degree of flexibility to enable the catheter to navigate tortuous paths within a body lumen, or the degree to which the catheter is capable of tracking a guidewire inserted therethrough. This is particularly important at the distal end of the catheter. The more flexible the distal portion of the catheter, the better the trackability.

Preferably, the or each of said inner hypotube and said outer hypotube is provided with a spiral cut profile that varies over the length of the hypotube. This allows the hypotubes to have different degrees of flexibility at each end. Ideally, each of said inner hypotube and said outer hypotube is provided with a spiral cut having a smaller pitch at its distal end and a larger pitch at its proximal end. The pitch of the spiral cut may be defined as the axial distance between adjacent turns of the spiral cut. At the distal end, the turns of the spiral cut are preferably of the order of between 0.1 and 10 mm apart, whereas at the proximal end, the turns of the spiral cut are preferably of the order of between 5 and 1000 mm apart. Alternatively, there may be no spiral cut at the proximal end of the hypotube. It is desirable that the profile of the spiral cut is graduated such that it provides a smooth variation rather than an abrupt change in flexibility. A sharp change in flexibility can render catheter hypotubes prone to kinking.

The flexibility imparted by the spiral cuts to the catheter, and in particular to the distal end of the catheter, allows the catheter to be manoeuvred or tracked through particularly tortuous paths of the vasculature. The closer the pitch of the spiral cut, the more flexible the hypotube. Decreasing the pitch at the distal end of each hypotube imparts more flexibility to the distal end, where flexibility and trackability are more important. At the proximal end of the catheter, where pushability is more important, the pitch of the spiral cut is increased and the hypotubes are thus relatively stiffer.

According to a preferred feature of the invention, the spiral cut in the or each hypotube is discontinuous, such that struts are provided where a portion of the circumference of the tube remains uncut.

The use of slots and the intermittent spiral or circumferential cut profile allows excellent pushability and flexibility. It reduces the compression and stretching of the shafts during the procedure. Any compression or stretching of the shaft can affect the position of the stent during the deployment process.

An advantage of the struts is to improve the pushability of the hypotubes through the anatomy of the patient. Pushability is a measure of the transfer of the force from the proximal end to the distal end of the assembly, that is, the ratio of force applied at the proximal end by the physician as compared to the force measured at the distal end. Good pushability means that force is efficiently and effectively transferred through the assembly.

A further purpose of the struts is to prevent stretching and compression of the spiral cut hypotubes while under stress. It is very important that the inner and outer hypotubes do not stretch or compress while being positioned within the vasculature. It is of particular importance that the outer tube is not allowed to stretch when being retracted during stent deployment. If the outer tube stretches rather than moving in a proximal direction during retraction, the stent will not be deployed correctly. The outer tube should be capable of clean retraction from the body lumen to allow the stent to correct the stenosis of the lumen. The strut-spiral combination is capable of withstanding stretching and compression under stress. This allows the catheter assembly to hold its position during stent deployment and thus allows more accurate placement of the stent during the delivery procedure. The desired strut length will depend on the pushability requirements for a given catheter, but will generally be of the order of between 0.1 mm and 1.0 mm.

Ideally, the struts are staggered around the circumference of the hypotube. For example, two struts may be provided on each spiral revolution, that is, substantially diametrically opposed about the circumference of the hypotube. Struts may be located between 90° and 270° from each other. Staggering the struts around the circumference of the hypotubes ensures adequate flexibility in all directions.

Preferably the outer and inner hypotubes are covered with a polymer jacket. The jacket is typically a fluropolymer, nylon or polyester type material. It can be applied using a variety of techniques known to the industry such as heat shrinking, over extrusions, heat bonding, chemical bonding or the like. The purpose of this jacket is to seal the outer hypotube and provide a sealed lumen to allow flushing of liquid through the device. Dependent on the characteristics of the device and the means of application, the jacket may also help to reduce stretching and compression of the hypotube shafts under an applied load. Additionally the outer shaft has the benefit of providing a smooth outer profile for the entire device, which has the benefit of reducing the risk of thrombosis while using this device in the vasculature.

Preferably, the stent is placed in the distal end of the device and makes contact with the inner diameter of the outer hypotube. This surface can be polished through a variety of means to reduce the friction between the stent and the outer shaft. Reducing the friction in this way will reduce the force needed to retract the outer shaft from the stent and subsequently make deployment of the stent easier. According to an optional feature the inner diametrical wall of the outer hypotube may be coated with low friction coating or have a low friction liner attached. Low friction liners are typically made from fluropolymer materials and can be bonded to the inside diameter of the outer hypotube using a variety of methods known in the industry such a chemical bonding, heat bonding and the like.

The stent can also be placed in a low friction outer sheath that is subsequently attached to the distal end of the device.

Further, a low friction jacket can be applied to the outer diameter of the outer hypotube. This jacket can be extended over the distal end of the device to a length suitable to enable the stent to be located in the inner diameter of this jacket but distal to the outer hypotube. Outer jackets of this type are typically made from fluropolymer materials and can be applied with a variety of methods known in the industry. In some cases this low friction coating can be a composite material or be braid reinforced. In one embodiment, the jacket can extend the full length of the device or alternatively over a shorter length at the distal end of the device. In the extreme case this outer jacket can be bonded to the distal end of the hypotube resulting in no overlap between the outer hypotube and the jacket material in which the stent is placed.

### Brief Description of the Drawings

Figure 1 is an elevation of a stent delivery catheter according to a preferred embodiment of the invention;
Figure 2 is a detailed view of the distal end of the stent delivery catheter of Figure 1, partly in cross-section;
Figure 3 is a detailed view of the distal end of the inner tube of the stent delivery catheter of Figure 1;
Figure 4 is a part sectional elevation of part of the wall of the inner tube of the stent delivery catheter shown in Figure 4, to an enlarged scale;
Figure 5 is an elevational view of the stent stop of the stent delivery catheter shown in Figure 3;
Figure 6 is an elevational view of the distal soft tip of the stent delivery catheter of Figure 1;
Figure 7 is a side elevational view of the wall of the outer tube of the stent delivery catheter of Figure 1 showing spiral cuts in the wall;
Figures 8 and 9 are cross-sectional views of alternative embodiments of the outer shaft of the stent delivery catheter of Figure 1;
Figure 10 is a perspective view of a portion of the spiral-cut hypotube of the stent delivery catheter of Figure 1, showing the spiral cut patterns;
Figure 11 is a perspective view similar to that of Figure 10, showing details of the spiral cut and intermediate struts;
Figure 12 is a plan view of portion of the hypotube, as shown in Figure 11, in a laid flat state to facilitate understanding of the configuration of the cuts and struts;
Figure 13 is a perspective view of an alternative embodiment of the hypotube showing circumferential cuts in the tube;
Figure 14 is a view of the hypotube of Figure 13 in a laid flat state showing the configuration of the cuts and intermediate struts;
Figure 15 is an elevation of a braided tubular section for attachment to a distal end of the hypotube;
Figure 16 is a cross-sectional view of the braided tube of Figure 14, and
Figure 17 is a view, similar to that of Figure 7, showing a further embodiment of the stent delivery catheter.

### Detailed Description of the Drawings

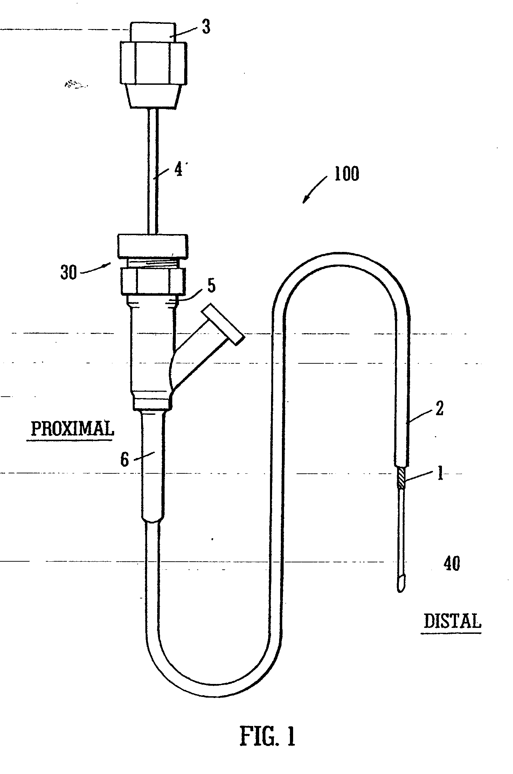

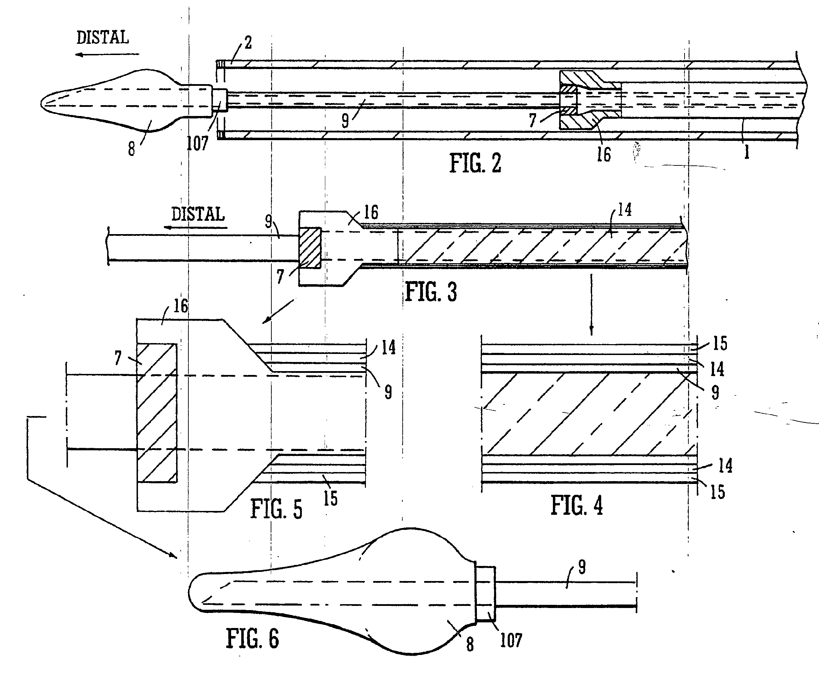

Figures 1 and 2 of the drawings show a stent delivery catheter assembly 100 according to the present invention. The catheter assembly has a proximal end generally designated 30 and a distal end generally designated 40. The assembly comprises an inner shaft or lumen 1 having proximal and distal ends concentrically located within an outer shaft or lumen 2. The outer shaft 2 extends beyond the distal end of the inner shaft 1, such that outer shaft extends in a distal direction beyond the distal end of said inner shaft. The distal end of said outer shaft is adapted to accommodate a stent for delivery within a body lumen of a patient. The catheter also comprises a guidewire lumen 9 having proximal and distal ends, which runs through the entire length of the catheter assembly within the inner shaft 1 and which extends beyond the distal end of the inner and outer shafts 1 and 2. The guidewire lumen 9 is attached to a guidewire hub 3 at the proximal end of the catheter. The proximal end 4 of the inner shaft 1 is stiffened relative to the distal end of the inner shaft.

The catheter further comprises a bifurcation luer 5.

The bifurcation luer 5 is a two ported luer, one port allowing access of a guidewire through the guidewire lumen, and the second port allowing flushing of the catheter system between the inner shaft and the outer shaft. A strain relief 6 provides stiffness transition between the bifurcation handle and the proximal outer shaft 2 to prevent kinking.

As shown in Figures 2 and 6, the guidewire lumen 9 is bonded at its distal end to a flexible tip 8. The tip 8 is formed from a relatively soft material and is flexible to allow for ease of tracking during the delivery procedure. The tip 8 is tapered such that it has a smaller diameter at its distal end, increasing to a larger diameter comparable to that of the outer shaft at its proximal end. The guidewire lumen 9 runs the entire length of the catheter assembly. During the delivery procedure, a guidewire is inserted within the guidewire lumen 9 and used to steer the tip of the catheter through the vasculature of the body to the site of stent deployment. The proximal portion of the guidewire lumen runs concentrically within the inner shaft 1 of the catheter assembly. The distal portion of the guidewire lumen 9 extends beyond the distal end of the inner shaft 1 within the outer shaft 2. This is the position at which the stent will be deployed.

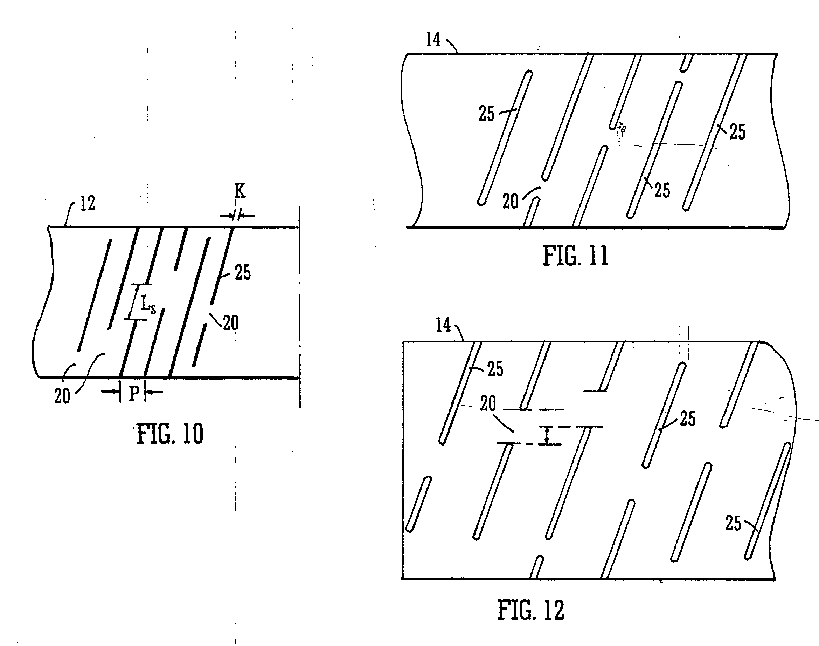

Referring now to Figures 2, 3, 4 and 5, the inner shaft 1 comprises a metallic spiral cut hypotube 14. Preferably, the metal is stainless steel but other suitable metals may be used. The hypotube 14 is spiral cut to allow for greater flexibility of the shaft in all directions. This gives the delivery system improved trackability, that is, it provides improved ability to navigate the tortuous passages of the human anatomy. One embodiment of the spiral cut is illustrated in more detail in Figures 10 to 12. The spiral cut hypotube 14 of the inner shaft 1 is covered by a polymer jacket 15. The purpose of the polymer jacket 15 is to provide a hermetic lumen during the flushing process through the bifurcation handle, (i.e. flush out air from the catheter system). The jacket also reduces the frictional properties of the shaft 1 therefore making relative movement of the inner shaft within the outer shaft of the catheter system easier. The jacket also contributes to reducing the stretching and compression of the shaft under an applied load. The jacket is typically a fluropolymer, nylon or polyester type material. It can be applied using a variety of techniques known to the industry such as heat shrinking, over extrusions, heat bonding, chemical bonding or the like.

The distal end of the spiral cut hypotube 14 is bonded to an inner shaft stop 16. The inner shaft stop 16 has an external diameter substantially the same as the inner diameter of the outer shaft 2. The inner shaft stop 16 acts as a mechanical proximal stop for the stent during the delivery procedure. The stent will be located within the outer shaft 2, distal to the inner shaft stop during delivery. The inner shaft stop 16 includes a two radio opaque markers 7 which are opaque to radio wave radiation. These markers may thus be used to determine the position of the proximal end of the stent during the delivery procedure. A further radio opaque marker 107 is provided at the distal end of the guidewire lumen 9, proximal to the tip 8. This marker indicates the position of the distal end of the stent during the delivery procedure.

Two markers, one each side of the stent are located on the distal end of the inner shaft. The marker bands extent the full circumference of the shaft. The proximal marker band is located in or distal to the stop 16, the distal marker band is located at the distal soft tip 18. The distance between the marker bands is representative of the length of the stent.

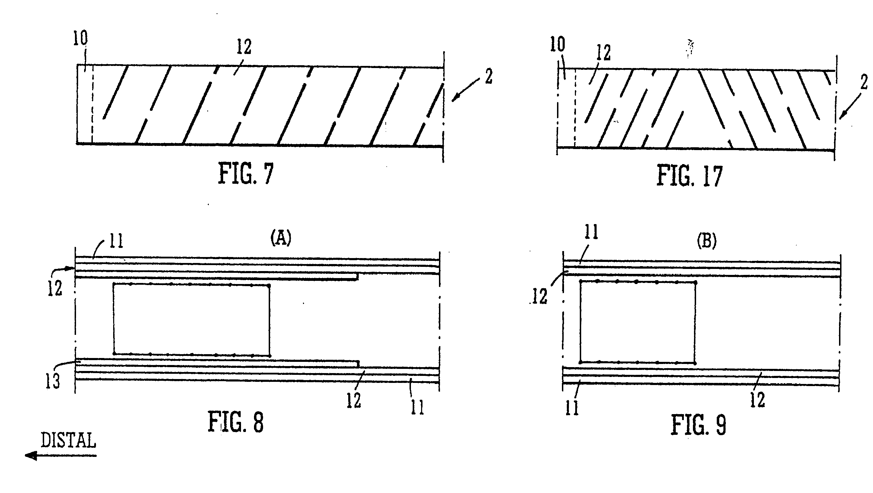

Figure 7 shows a detail of the distal portion of the outer shaft 2. The outer shaft comprises a metallic spiral cut hypotube 12. As with the inner spiral cut hypotube, the spiral cuts provide the catheter assembly with a high degree of flexibility and pushability. The outer shaft 2 has a further radio opaque marker band 10 at its distal end. Again, this may be used to determine the position of the catheter assembly during the delivery procedure. This marker band is also an indicator of the amount of stent remaining within the outer shaft 2 during the procedure. The outer shaft 2 also comprises an outer shaft polymer jacket 11, which runs the length of the outer shaft 2. As illustrated in Figures 8 and 9, the shaft may optionally further comprise a polymer lining sleeve 13. In the embodiment shown in Figure 8, the lining sleeve 13 runs along a distal portion of the internal wall of the outer hypotube 12, where the stent will be positioned prior to deployment.

Although not shown in the drawings the spiral cut may comprise a continuous uninterrupted slot 25 which follows a spiral path about the circumference of the hypotube, and continues for substantially its whole length.

However, a preferred configuration of the spiral cuts 25 in hypotubes 12 and 14 is shown in Figures 10 to 12. Each spiral-cut hypotube has a discontinuous cut formed in its outer wall, the cut extending spirally of the tube. The purpose of the spiral cuts 25 in the hypotubes is to add flexibility to the inner and outer shafts 1 and 2 The spiral cut is made completely through the wall thickness of the hypotube.

The spiral cut profile can be produced with a clockwise or counterclockwise rotation. A combination of clockwise and counterclockwise spiral on the same hypotube shaft is also a suitable configuration, one embodiment of which is shown in Figure 17. Alternatively, each individual slot 25 can extend in a path which is at an angle to the adjacent slot 25. That is one slot extends in a clockwise direction, and the adjacent slot extends in a counterclockwise direction to provide a zig-zag formation.

Alternatively, the cut 25 comprises scoring which serves to reduce the wall thickness of the hypotube in the area of the cut, but does not extend completely through the wall.

The flexibility imparted by the spiral cuts 25 to the distal end of the catheter in particular allows for improved tracking, that is, catheter may be more easily manoeuvred through particularly tortuous paths of the vasculature. The flexibility of the hypotube is dependent on the pitch p of the spiral cut, which is the axial distance between adjacent turns of the spiral cut, as shown in Figure 10. A variable spiral cut profile may be applied along the length of the shaft to give more flexibility at the distal end and less at the proximal end where it is not of great importance. For example, the pitch p at the distal end is preferably of the order of between 0.1 and 10 mm, whereas the pitch at the proximal end is preferably between 5 and 1000 mm. It is also possible to provide no spiral cuts at all at the proximal end of the hypotubes. The kerf width K, or width of each cut, is generally of the order of 0.01 - 1.0mm, preferably about 0.025mm. The angle of the spiral is typically between 30 and 90 degrees to the circumferential axis of the hypotube.

In the embodiment shown, the spiral cuts are provided with struts 20. The struts are areas where the spiral cut is discontinuous, that is, there is a section of the tube that remains intact.

One purpose of the struts 20 is to improve the pushability of the hypotubes through the body lumens of the patient. Pushability is a measure of the transfer of the force from the proximal end to the distal end of the assembly, that is, the ratio of force applied at the proximal end by the physician as compared to the force measured at the distal end. Good pushability means that force is efficiently and effectively transferred through the assembly. A further purpose of the struts is to prevent stretching and compression of the spiral cut hypotubes while under stress. The strut-spiral combination is capable of minimising stretching and compression under stress. This allows the catheter assembly to more accurately hold its position during stent deployment and thus allows more accurate placement of the stent during the delivery procedure. The strut length Lₛ will depend on the pushability requirements for a given catheter, but will generally be of the order of 0.1 mm to 1.0 mm. In the embodiment shown, two struts are provided on each spiral revolution, that is, substantially diametrically opposed about the circumference of the hypotube. Struts may be located between 90° and 270°from each other. Preferably the struts are staggered around the circumference of the hypotube to give adequate flexibility in all directions.

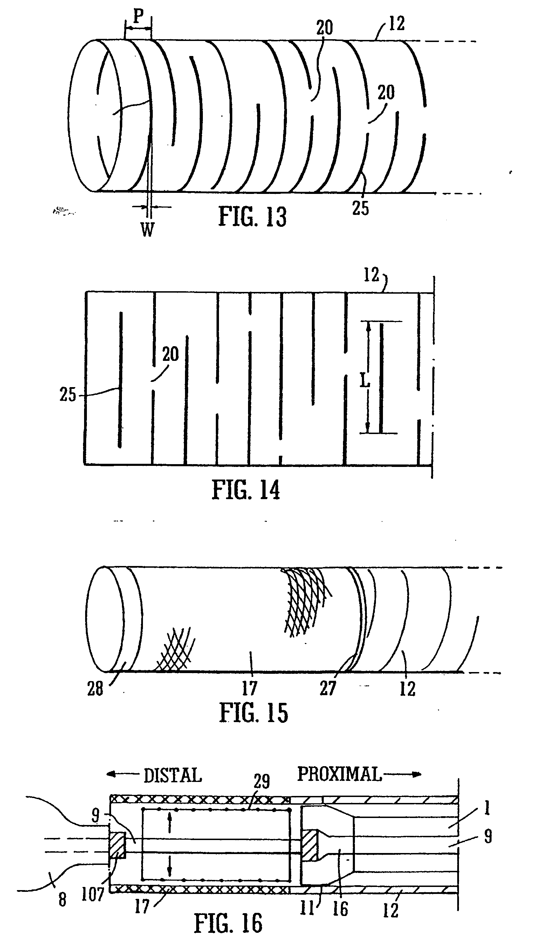

A further embodiment of a cut hypotube is shown in Figures 13 and 14. Figure 13 shows the tube in elevation whereas Figure 14 shows the wall section of the tube laid flat. In this embodiment the slots 25 extend about a substantially circumferential path. However, as with the previous embodiment of spiral cuts, the path is interrupted by solid struts 20. It will be noted that there is at least one slot 25 in each circumferential plane extending partly around the circumference of the hypotube 12 to define at least one strut in that plane. The struts 25 are staggered substantially in the same way as the spiral struts. That is, each slot 25 in one plane is located opposite a strut 20 in an adjacent plane.

The staggering of the slots 25 ensures flexibility in all planes. The pitch Pof the slots 25 is as described previously in relation to the spirally oriented slots The width W is larger in this embodiment and may vary between 0.1mm and 2mm The length L of the cut slot can vary depending on required shaft performance characteristics, the slot length generally varies between 20% and 80% of the tube circumference.

Figures 15 and 16 shows a braided hypotube section 17 which is attached, by chemical bonding (e.g. an adhesive) or heat bonding 27 to the distal end of the outer hypotube 12. The bond may be achieved by overlapping the proximal end of the braided hypotube section over the distal end of the outer hypotube 12. The braided hypotube shaft 17 comprises a stainless steel wire mesh braid sandwiched between two layers of polymer material. The stainless steel braid imparts rigidity and column strength to the shaft 17. Suitably, the inner polymer layer is comprised of a material of low frictional properties, such as a fluropolymer or a blend of fluropolymers. The outer polymer layer suitably is a polymer having good flex properties, such as nylon or Pebax. The distal end of the braided hypotube 17 is provided with a radio opaque marker band 28.

Figure 16 shows a section through Figure 15 and shows the position of the stent 29. The stent 29 is located, as is mentioned in previous embodiment, distal to the proximal stent placement marker band 7 and proximal to the distal stent marker band 107. The stent is constrained within the braided shaft 17. The braided shaft is bonded to the outer shaft 12 as mentioned above. The stop 16 is located within the inner diameter of the braided shaft 17 and serves as mechanical stop during deployment of the stent 29 during procedure, that is when outer shaft and hence the braided shaft 17 is retracted in proximal direction from the lumen effecting stent deployment.

The slots 25 may be cut in the wall of the hypotubes by a variety of techniques. A preferred method is by laser cutting but other techniques could be used such as chemical etching, diamond etching , mechanical or chemical cutting.

Before use, a self-expanding stent 29 is compressed and inserted into the distal end of the outer shaft 2 (see Fig. 16). The inner diameter of the outer shaft is such that the stent is held in a compressed position to give it a reduced diameter for delivery purposes. The delivery catheter assembly and stent are advanced through the inner lumens of the body to the site of desired stent deployment, for example, a site of stenosis of a blood vessel. The guidewire and the flexible tip are used to steer the assembly though the anatomy of the body to the body lumen where the stent is to be deployed. The spiral-cut hypotubes are sufficiently flexible to allow the assembly to be manoeuvred or tracked through the tortuous pathways of the anatomy of the body. The struts provide the assembly with good pushability such that the force applied by a physician at the proximal end of the assembly is effectively transferred to the tip of the catheter. The radio opaque markers 7, 107 and 10 may be used to monitor the position of the catheter and the stent accommodated therein and to determine when the stent is in the correct position. The proximal end of the stent abuts the inner shaft stop 16. When the stent has been correctly located, the outer shaft is withdrawn from the body. The inner shaft 1 remains in place within the body lumen and the stop 16 prevents the stent from being withdrawn with the outer shaft. As the outer shaft is withdrawn, the compressive force on the stent is removed and the stent expands to its original unstressed position. This expansion exerts an outward radial force on the constricted body lumen, counteracting the constriction and widening the lumen. Patency is thus restored to the body lumen.

## Claims

1. A delivery catheter assembly (100) for a self-expanding stent or other device deployed in a similar fashion, comprising an inner shaft (1), having proximal and distal ends, and an outer shaft (2), having proximal and distal ends, and the inner shaft is located coaxially within said outer shaft, and said outer shaft extends beyond the distal end of said inner shaft, said distal end of said outer shaft being adapted to accommodate the stent for delivery within a body lumen of a patient, **characterised in that** the inner shaft (1) is an elongate metallic hypotube (14) and the outer shaft (2) is an elongate metallic hypotube (12), and each of the hypotubes (12, 14) has at least one cut (25) in the side wall thereof to increase the flexibility of the hypotube, and the or each cut is an elongate cut (25) extending about the hypotube in a spiral or circumferential path which is interrupted by at least one solid strut (20).

2. An assembly as claimed in claim 1, **characterised in that** the cut or cuts extend completely through the wall thickness of each hypotube.

3. An assembly as claimed in claim 1, **characterised in that** the cut or cuts comprise a score or scores formed in the outer surface of the wall of the hypotubes.

4. An assembly as claimed in any one of the preceding claims, **characterised in that** each hypotube (12, 14) is provided with a spiral cut profile that extends in a spiral path around the hypotube (12, 14).

5. An assembly as claimed in any preceding claim, **characterised in that** the or each hypotube is provided with a spiral cut having a smaller pitch at its distal end and a larger pitch at its proximal end.

6. An assembly as claimed in claim 4 or claim 5, **characterised in that** the spiral cut in each hypotube (12, 14) is discontinuous, such that struts (20) are provided where a portion of the circumference of the tube remains uncut.

7. An assembly as claimed in claim 6, **characterised in that** the struts (20) are staggered around the circumference of the hypotube (12,14).

8. An assembly as claimed in claim 1, **characterised in that** the cut or cuts are in the form of a slot or slots (25) cut through the outer wall of the or each hypotube (12,14) and each slot (25) extends substantially circumferentially around part of the circumference of the hypotube; there being at least one slot (25) in each circumferential plane extending partly around the circumference of the hypotube (12,14) to define at least one strut (20) **in that** plane.

9. An assembly as claimed in claim 8, **characterised in that** a plurality of slots (25) are formed in the wall of the hypotube in substantially parallel arrangement axially of the hypotube.

10. An assembly as claimed in claim 8 or claim 9 **characterised in that** at least one strut (20) is positioned on each circumferential revolution of the cuts, and the struts (20) in one revolution are in staggered relationship to the struts (20) in adjacent revolutions or circumferential planes.

11. A catheter assembly as claimed in any of the preceding claims **characterised in that** the inner wall of the outer shaft (2) is coated with a low friction coating or has attached thereto a low friction liner (13).

12. A delivery catheter assembly as claimed in any preceding claim, **characterised in that** the stent is constrained within a braided shaft (17) which is attached to the distal end of the outer shaft (12).

13. A catheter assembly as claimed in any preceding claim, **characterised in that** the outer shaft comprises an outer polymer jacket (15) allowing the shaft (12, 14) maintain a hermetic lumen,

## Patentansprüche

1. Einführkatheteranordnung (100) für einen selbstexpandierenden Stent oder eine andere Vorrichtung, die sich auf im Wesentlichen gleiche Weise entfaltet, mit einem Innenschaft (1) mit proximalen und distalen Enden und einem Außenschaft (2) mit proximalen und distalen Enden, wobei der Innenschaft koaxial in dem Außenschaft angeordnet ist, der Außenschaft über das distale Ende des Innenschafts hinaus verläuft und das distale Ende des Außenschafts zum Aufnehmen des zum Einführen in ein Körperlumen des Patienten bestimmten Stent vorgesehen ist,
**dadurch gekennzeichnet, dass**
der Innenschaft (1) ein längliches metallenes Hypotube (14) ist und der Außenschaft (2) ein längliches metallenes Hypotube (12) ist und jedes Hypotube (12,14) mindestens einen Einschnitt (25) zum Erhöhen der Flexibilität des Hypotube in seiner Seitenwand aufweist und der oder jeder Einschnitt ein länglicher Einschnitt (25) ist, der in einem spiralförmigen oder umfangsmäßigen Weg um das Hypotube verläuft, wobei der Weg an mindestens einem massiven Steg (20) unterbrochen ist.

2. Anordnung nach Anspruch 1, **dadurch gekennzeichnet, dass** der Einschnitt oder die Einschnitte vollständig die Wanddicke jedes Hypotube durchlaufen.

3. Anordnung nach Anspruch 1, **dadurch gekennzeichnet, dass** der Einschnitt oder die Einschnitte eine Kerbe oder Kerben in der Außenfläche der Wand des Hypotube aufweisen.

4. Anordnung nach einem der vorhergehenden Ansprüche, **dadurch gekennzeichnet, dass** jedes Hypotube (12,14) ein spiralförmiges Einschnittprofil aufweist, das in einem spiralförmigen Weg um das Hypotube (12,14) verläuft.

5. Anordnung nach einem der vorhergehenden Ansprüche, **dadurch gekennzeichnet, dass** das oder jedes Hypotube einen spiralförmigen Einschnitt mit einer kleinere Steigung an seinem distalen Ende und einer größeren Steigung an seinem proximalen Ende aufweist.

6. Anordnung nach Anspruch 4 oder 5, **dadurch gekennzeichnet, dass** der spiralförmige Einschnitt in jedem Hypotube (12,14) derart diskontinuierlich ist, dass Stege (20) dort vorgesehen sind, wo ein Teil des Umfangs des Tube keine Einschnitte aufweist.

7. Anordnung nach Anspruch 6, **dadurch gekennzeichnet, dass** die Stege (20) um den Umfang des Hypotube (12,14) versetzt angeordnet sind.

8. Anordnung nach Anspruch 1, **dadurch gekennzeichnet, dass** der Einschnitt oder die Einschnitte in der Form eines Schlitzes oder von Schlitzen (25) ausgebildet sind, die in die Außenwand des oder jedes Hypotube (12,14) geschnitten sind, und jeder Schlitz (25) im Wesentlichen um einen Teil des Umfangs des Hypotube verläuft; wobei mindestens ein Schlitz (25) in jeder Umfangsebene teilweise um den Umfang des Hypotube (12,14) verläuft, um mindestens einen Steg (20) in dieser Ebene zu begrenzen.

9. Anordnung nach Anspruch 8, **dadurch gekennzeichnet**, das mehrere Schlitze (25) in der Wand des Hypotube im Wesentlichen parallel zueinander in Axialrichtung des Hypotube ausgebildet sind.

10. Anordnung nach Anspruch 8 oder 9, **dadurch gekennzeichnet, dass** mindestens ein Steg (20) an jedem Umfangsumlauf der Einschnitte positioniert ist und die Stege (20) in einem Umlauf zu den Stegen (20) in benachbarten Umläufen oder benachbarten Umfangsebenen versetzt angeordnet sind.

11. Katheteranordnung nach einem der vorhergehenden Ansprüche, **dadurch gekennzeichnet, dass** die Innenwand des Außenschafts (2) mit einer reibungsarmen Beschichtung beschichtet ist oder mit einer reibungsarmen Auskleidung (13) versehen ist.

12. Einführkatheteranordnung nach einem der vorhergehenden Ansprüche, **dadurch gekennzeichnet, dass** der Stent in einem umflochtenen Schaft (17) festgehalten ist, der an dem distalen Ende des Außenschafts (2) angebracht ist.

13. Katheteranordnung nach einem der vorhergehenden Ansprüche, **dadurch gekennzeichnet, dass** der Außenschaft eine Polymer-Außenummantelung (15) aufweist, durch die der Schaft (12,14) ein hermetisches Lumen aufrechterhalten kann.

## Revendications

1. Ensemble cathéter (100) de déploiement pour une endoprothèse auto-déployable ou un autre dispositif déployé d'une façon similaire, comprenant une tige interne (1), ayant une extrémité proximale et une extrémité distale, et une tige externe (2), ayant une extrémité proximale et une extrémité distale, et la tige interne est située de façon coaxiale à l'intérieur de ladite tige externe, et ladite tige externe s'étend au-delà de l'extrémité distale de ladite tige interne, ladite extrémité distale de ladite tige externe étant adaptée pour accueillir l'endoprothèse pour un déploiement à l'intérieur d'une lumière de corps d'un patient, **caractérisé en ce que** la tige interne (1) est un hypotube métallique allongé (14) et la tige externe (2) est un hypotube métallique allongé (12), et chacun des hypotubes (12, 14) a au moins une coupe (25) dans la paroi latérale de celui-ci pour augmenter la flexibilité de l'hypotube, et la coupe ou chaque coupe est une coupe allongée (25) s'étendant autour de l'hypotube dans une trajectoire spiroïdale ou circonférentielle qui est interrompue par au moins une entretoise solide (20).

2. Ensemble selon la revendication 1, **caractérisé en ce que** la coupe ou les coupes s'étendent complètement à travers l'épaisseur de paroi de chaque hypotube.

3. Ensemble selon la revendication 1, **caractérisé en ce que** la coupe ou les coupes comprennent une rainure ou des rainures formées dans la surface externe de la paroi des hypotubes.

4. Ensemble selon l'une quelconque des revendications précédentes, **caractérisé en ce que** chaque hypotube (12, 14) est muni d'un profil de coupe spiroïdale qui s'étend dans une trajectoire spiroïdale autour de l'hypotube (12, 14).

5. Ensemble selon l'une quelconque revendication précédente, **caractérisé en ce que** l'hypotube ou chaque hypotube est muni d'une coupe spiroïdale ayant un plus petit pas au niveau de son extrémité distale et un plus grand pas au niveau de son extrémité proximale.

6. Ensemble selon la revendication 4 ou la revendication 5, **caractérisé en ce que** la coupe spiroïdale dans chaque hypotube (12, 14) est discontinue, de telle sorte que des entretoises (20) sont fournies à l'endroit où une partie de la circonférence du tube reste non coupée.

7. Ensemble selon la revendication 6, **caractérisé en ce que** les entretoises (20) sont étagées autour de la circonférence de l'hypotube (12, 14).

8. Ensemble selon la revendication 1, **caractérisé en ce que** la coupe ou les coupes se trouvent sous la forme d'une fente ou de fentes (25) coupées à travers la paroi externe de l'hypotube ou de chaque hypotube (12, 14) et chaque fente (25) s'étend de façon sensiblement circonférentielle autour de la partie de la circonférence de l'hypotube, au moins une fente (25) se trouvant dans chaque plan circonférentiel s'étendant partiellement autour de la circonférence de l'hypotube (12, 14) pour définir au moins une entretoise (20) dans ce plan.

9. Ensemble selon la revendication 8, **caractérisé en ce qu'**une pluralité de fentes (25) est formée dans la paroi de l'hypotube dans une disposition sensiblement parallèle de façon axiale de l'hypotube.

10. Ensemble selon la revendication 8 ou la revendication 9, **caractérisé en ce que** au moins une entretoise (20) est positionnée sur chaque révolution circonférentielle des coupes, et les entretoises (20) dans une révolution se trouvent dans une relation étagée avec les entretoises (20) dans des révolutions adjacentes ou dans des plans circonférentiels.

11. Ensemble cathéter selon l'une quelconque des revendications précédentes, **caractérisé en ce que** la paroi interne de la tige externe (2) est revêtue d'un revêtement à faible frottement ou a fixé sur celle-ci une enveloppe (13) à faible frottement.

12. Ensemble cathéter de déploiement selon l'une quelconque revendication précédente, **caractérisé en ce que** l'endoprothèse est maintenue à l'intérieur d'une tige tressée (17) qui est fixé sur l'extrémité distale de la tige externe (12).

13. Ensemble cathéter selon l'une quelconque revendication précédente, **caractérisé en ce que** la tige externe comprend une chemise externe (15) en polymère permettant à la tige (12, 14) de maintenir une lumière hermétique.
